# EUROPÄISCHE PATENTSCHRIFT

(11) **EP 2 039 545 B1**
(45) Veröffentlichungstag und Bekanntmachung des Hinweises auf die Patenterteilung: **31.03.2010**
(21) Anmeldenummer: 08016548.3
(22) Anmeldetag: 19.09.2008
(51) Int. Cl.: B60H 3/00

(54) **Beduftungssystem, insbesondere für ein Kraftfahrzeug**
Fragrance system, in particular for a motor vehicle
Système de parfum, en particulier pour un véhicule automobile

(30) Priorität: 20.09.2007 DE 102007045160
(43) Veröffentlichungstag der Anmeldung: 25.03.2009
(73) Patentinhaber: Behr GmbH & Co. KG, 70469 Stuttgart (DE)
(72) Erfinder: Wolf, Walter, 71570 Oppenweiler-Zell (DE)

(56) Entgegenhaltungen:
- EP-A- 1 323 556
- DE-A1- 10 327 122
- US-A- 4 808 347
- US-A- 5 314 669

## Beschreibung

Die Erfindung betrifft ein Beduftungssystem gemäß dem Oberbegriff des Anspruches 1.

Aus der DE 103 27 122 A1, der als nächstliegender Stand der Technik angesehen wird, ist eine Belüftungsvorrichtung, insbesondere zum Beduften eines Kraftfahrzeugs, bekannt, welche einen Duftstoff enthält, mit einer Dosiereinrichtung, wobei die Beduftungsvorrichtung zumindest einen Hohlkörper, der mit mindestens einer Öffnung versehen ist, und ein Element, insbesondere einen Hohlkörper, mit mindestens einer weiteren Öffnung umfasst. Dabei ist der Hohlkörper und/oder das Element mittels eines Motors derart antreibbar, dass eine Relativbewegung zwischen dem Hohlkörper und dem Element möglich ist, so dass die beiden Öffnungen in zumindest einer Stellung der Relativbewegung ein Austreten von Duftstoff von innen nach außen ermöglichen. Der Duftstoff selbst ist innerhalb des Hohlkörpers in einer Duftstoffpatrone oder -kartusche angeordnet, die bei Bedarf auswechselbar ist.

Eine andere Beduftungsvorrichtung ist aus der US 5,314,669 A bekannt. Bei dieser Beduftungsvorrichtung sind zwei in axialer Richtung zueinander angeordnete Hohlzylinder in einem im Wesentlichen als Hohlzylinder ausgebildeten Gehäuse drehbar aufgenommen. Der das Gehäuse bildende Hohlzylinder weist zwei rechteckförmige, sich in Längsrichtung erstreckenden Öffnungen in seiner Mantelfläche auf einander gegenüberliegenden Seiten auf, die sich jeweils zumindest über einen Teilbereich der inneren Hohlkörper erstrecken. Die inneren Hohlzylinder weisen ebenfalls jeweils zwei einander gegenüberliegende Öffnungen in ihren Mantelflächen auf, die jedoch gestuft ausgebildet sind, d.h. die Form dreier jeweils um die Hälfte ihrer Länge versetzt zueinander angeordneter, langgestreckter und zusammen eine gemeinsame Öffnung bildender Rechtecke aufweisen. Die Öffnungen der inneren Hohlzylinder sind verdreht zueinander angeordnet, so dass verschiedene Beduftungsvariationen möglich sind.

Die US 5,178,327 A offenbart ein Beduftungssystem mit einem in mehrere Sektoren unterteilten, drehbar in einem Gehäuse angeordneten Hohlzylinder, wobei mindestens ein kuchenartiger Teil des Hohlzylinders in der Größe eines Segments fehlt, weshalb auf den Hohlzylinder im Folgenden als Teil-Hohlzylinder Bezug genommen wird. Die Segmente sind jeweils stirnseitig offen und in jedem der Segmente ist ein anderer Duftstoff eingelagert. Im Gehäuse ist auf einer oder beiden Seiten in axialer Richtung des Teil-Hohlzylinders eine in Ihrer Gestalt im Wesentlichen dem Querschnitt eines Segments entsprechende Öffnung ausgebildet. Um einen Raum zu beduften, wird das den gewünschten Duft enthaltende Segment vor die Öffnung gedreht. Wird keine Beduftung gewünscht, so wird das fehlende Segment vor die Öffnung gedreht. Um eine stärkere Beduftung eines Raumes zu ermöglichen, kann auch ein Lüfter vorgesehen sein, welches Luft durch die erste Öffnung in das Gehäuse und nach dem Durchströmen des Teil-Hohlzylinders durch die zweite, gegenüberliegende Öffnung dem zu beduftenden Raum zuführt.

Ferner ist aus der nachveröffentlichten EP 1 854 647 A1 ein Beduftungssystem bekannt, mit zumindest einem ersten Bauteil und zweiten Bauteil, welches das zumindest erste Bauteil im Wesentlichen umgreift oder von diesem umgriffen wird, wobei das zumindest eine erste und das zumindest eine zweite Bauteil jeweils eine prismatische Innen- beziehungsweise Außenmantelfläche aufweisen, die auch unterbrochen ausgebildet sein kann, und relativ zueinander bewegbar sind, in einem der Bauteile mindestens zwei Aufnahmekammern ggf. mit Duftstoff enthalten sind, und durch das zumindest eine, erste Bauteil und das zumindest eine, zweite Bauteil in Abhängigkeit der Position zueinander mindestens eine Verbindung zur Umgebung freigeben ist, über welche bei Bedarf Duftstoff nach außen aus der oder den Aufnahmekammern abgegeben werden kann. Die Aufnahmekammern sind hierbei durch zumindest eine in Längsrichtung des Bauteils verlaufende Trennwand voneinander getrennt, und die Verbindung zur Umgebung vorgesehen ist über zumindest eine in einer prismatischen Mantelfläche des Bauteils, in welchem die Aufnahmekammern für den oder die Duftstoffe angeordnet sind, ausgebildete Öffnung. Die beiden Bauteile können hierbei durch koaxial angeordnete Hohlzylinder gebildet sein. Der erste, innere Hohlzylinder ist mittels eines Antriebs, bspw. eines elektrischen Schrittstellmotors, drehbar in einen eine Umschließung bildenden zweiten äußeren Hohlzylinder angeordnet, wobei ein Ringspalt zwischen der Außenmantelfläche des inneren Hohlzylinders und der Innenmantelfläche des äußeren Hohlzylinders ausgebildet ist, der durch elastische Dichtelemente unterbrochen ist, die an der Außenmantelfläche des inneren Hohlzylinders in Verlängerung der Trennwände, welche die Aufnahmekammern unterteilen, angeordnet sind. Der äußere Hohlzylinder ist fest mit dem Gehäuse des Antriebs verbunden, während der innere Hohlzylinder mit der Abtriebswelle desselben verbunden ist, so dass eine Relativbewegung der Hohlzylinder zueinander möglich ist. Das Gebläse einer Klimaanlage kann für eine ausreichende Luftdurchströmung sorgen, so dass der gewünschte Duftstoff dem Fahrzeuginnenraum zugeführt wird.

Derartige Beduftungssysteme lassen noch Wünsche offen.

Es ist Aufgabe der Erfindung, ein verbessertes Beduftungssystem zur Verfügung zu stellen.

Diese Aufgabe wird gelöst durch ein Beduftungssystem mit den Merkmalen des Anspruchs 1. Vorteilhafte Ausgestaltungen sind Gegenstand der Unteransprüche.

Hierbei ist ein Beduftungssystem, insbesondere für ein Kraftfahrzeug, vorgesehen, aufweisend mindestens zwei relativ zueinander verdrehbare Bauteile, wobei in mindestens einer Relativstellung der Bauteile zueinander mindestens eine Öffnung freigegeben ist, welche in mindestens einer anderen Relativstellung der Bauteile verschlossen ist, und mindestens ein von einem Motor antreibbares Lüfterrad eines Gebläses, welches auf der Motorwelle sitzt oder mit derselben gekoppelt ist, wobei mindestens ein Freilauf zwischen der Motorwelle und den relativ zueinander verdrehbaren Bauteilen des Beduftungssystems vorgesehen ist. Durch das Vorsehen eines Freilaufs kann die Drehbewegung des Motors im Falle einer Drehrichtungsumkehr für eine Stellbewegung der beiden relativ zueinander verdrehbaren Bauteile benutzt werden, so dass die Öffnung entweder in Folge der Stellbewegung freigegeben oder verschlossen wird. Die Betätigung kann somit einfach über die Steuerung des Motors erfolgen und direkt in das allgemeine System eines Fahrzeugs, welches zur Überwachung der Temperatur sowie ggf. weiterer Parameter im Fahrzeuginnenraum dient, integriert werden.

Als Bauteile sind bevorzugt ein erstes, im Wesentlichen hohl ausgebildetes Bauteil, welches mindestens eine Aufnahmekammer für einen Duft oder eine einen Duft enthaltende Patrone bildet, und als zweites Bauteil ein das erste Bauteil zumindest bereichsweise umgebendes Gehäuse vorgesehen, wobei zwischen den beiden Bauteilen mindestens ein Luftkanal ausgebildet ist, welcher Luft zumindest bereichsweise in das hohl ausgebildete Bauteil einund ausleitet oder an einer Öffnung desselben vorbeileitet, so dass der Luftstrom im Falle einer Beduftung in Kontakt mit dem entsprechenden Duft gelangt und gemeinsam mit demselben in den Fahrzeuginnenraum gelangt.

Der Luftkanal weist bevorzugt einen zwischen den beiden Bauteilen angeordneten Querschnitt auf, welcher sich über den Strömungsweg der Luft zuerst verkleinert und nach Erreichen eines Minimalquerschnitts, welcher auch Null sein kann, wieder vergrößert. Hierbei sind in dem Bereich besagte Öffnungen im ersten Bauteil vorhanden, durch welche die Luft in das erste Bauteil einströmt und - nach Aufnahme des entsprechenden Dufts - wieder ausströmt, d.h. der Luftkanal wird bereichsweise in das erste Bauteil verlagert.

Bevorzugt ist eine starre Koppelung zwischen Motor und einem Lüfterrad des Gebläses vorgesehen, wobei sich die Drehrichtung des Lüfterrades im Falle einer Stellbewegung der beiden relativ zueinander verdrehbaren Bauteile umdreht.

Zwischen dem Motor und einem Lüfterrad des Gebläses kann ein zweiter Freilauf vorgesehen sein, welcher in entgegengesetzter Richtungen zum ersten Freilauf eine Relativbewegung freigibt oder das Lüfterrad an die Welle, insbesondere Motorwelle, ankoppelt.

Besonders bevorzugt ist der Motor direkt in das Gebläse integriert. Hierbei kann der Stator im Gehäuse um das Lüfterrad des Gebläses angeordnet sein. Der Rotor kann direkt in das Lüfterrad integriert sein.

Das erste Bauteil, welches relativ zu einem zweiten Bauteil verdrehbar ist, ist besonders bevorzugt starr, insbesondere bevorzugt einstückig, mit einem abtriebsseitigen Teil des Freilaufs verbunden.

Bevorzugt ist im abtriebsseitigen Teil des Freilaufs der antriebsseitige Teil des Freilaufs konzentrisch drehbar gelagert. Hierfür kann bspw. eine Lagerbuchse eingegossen sein.

Im Folgenden wird die Erfindung anhand von Ausführungsbeispielen, teilweise unter Bezugnahme auf die Zeichnung, im Einzelnen erläutert. Es zeigen:

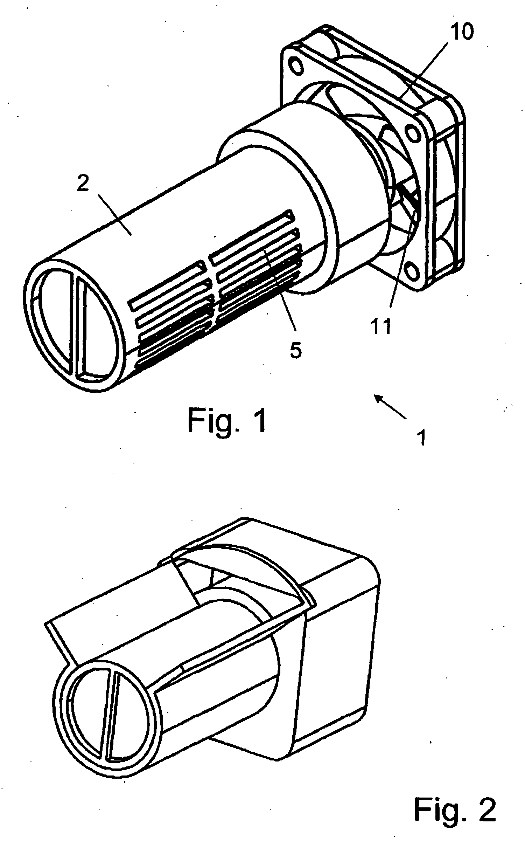

- Fig. 1: eine perspektivische Ansicht eines Beduftungssystems ohne Darstellung des Gehäuses gemäß dem ersten Ausführungsbeispiel,
- Fig. 2: eine Fig. 1 entsprechende Darstellung des Beduftungssystems samt Gehäuse in der Stellung "zu" ohne Darstellung Blende im Bereich der Luftein- und -ausleitung in und aus der entsprechenden Aufnahmekammer für einen Duft,
- Fig. 3: eine andere perspektivische Ansicht des Beduftungssystems von Fig. 2 mit Darstellung der Blende,
- Fig. 4: eine weitere perspektivische Ansicht des Beduftungssystems von Fig. 2,
- Fig. 5: eine schematische Schnittdarstellung des Beduftungssystems an der Engstelle in der Stellung "zu",

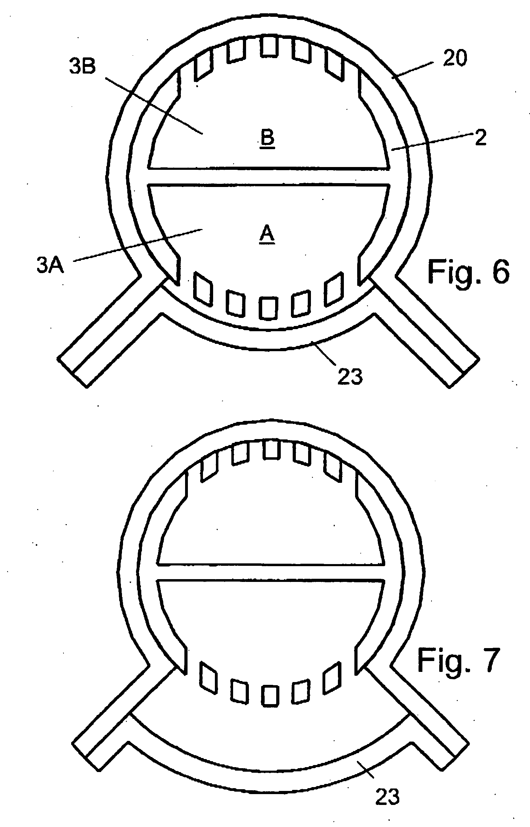

- Fig. 6: eine Fig. 5 entsprechende Schnittdarstellung des Beduftungssystems an der Engstelle in der Stellung "auf",
- Fig. 7: eine schematische Schnittdarstellung des Beduftungssystems an der Lufteinströmstelle in der Stellung "auf",
- Fig. 8a: eine schematische Darstellung des Freilaufs mit einer durch den Pfeil angedeuteten Wellendrehrichtung "Klemmen",
- Fig. 8b: eine schematische Darstellung des Freilaufs mit einer durch den Pfeil angedeuteten Wellendrehrichtung "Freilauf",
- Fig. 9: einen schematisch dargestellten Längsschnitt durch das Beduftungssystem von Fig. 6 samt Freilauf und Andeutung des Luftstroms durch Pfeile, und
- Fig. 10: eine perspektivische Ansicht der Duftpatrone samt Dichtelement und Freilauf und einem Dichtelement gemäß dem dritten Ausführungsbeispiel.

Ein zentral auf einer Instrumententafel eines Kraftfahrzeugs angeordnetes Beduftungssystem 1 gemäß dem ersten Ausführungsbeispiel weist einen inneren Hohlzylinders 2 mit vorliegend zwei Duftreservoirs oder Aufnahmekammern 3 auf, vorliegend die mit einem ersten Duft A gefüllte Aufnahmekammer 3A und die mit einem zweiten Duft B gefüllte Aufnahmekammer 3B. Die einzelnen Aufnahmekammern 3A, 3B sind mittels einer mittig angeordneten Trennwand voneinander getrennt, die Teil des inneren Hohlzylinders 2 ist, wobei vorliegend die Aufnahmekammern jeweils gleich groß sind. Jeder der Aufnahmekammern weist zwei Reihen sich von über einen Teil des Umfangs erstreckende Schlitze zur Bildung von Ein- und Austrittsöffnungen 5 auf, durch welchen der Duft A oder B aus der entsprechenden Aufnahmekammer 3A bzw. 3B austreten kann. Der innere Hohlzylinder ist oben und unten durch Wände verschlossen.

Um unabhängig von der Belüftung des Fahrzeugs oder sonstigen Gegebenheiten, wie Luftbewegung im Innenraum, konstant den gewünschten Duft dem Fahrzeuginnenraum zuführen zu können, ist an einem Ende ein Gebläse 10 mit einem Lüfterrad 11 vorgesehen, welches gleichzeitig den Rotor eines Motors 12 bildet, dessen Stator um das Lüfterrad 11 angeordnet ist, so dass die Lüfterradwelle gleichzeitig auch die Motorwelle ist und im Folgenden kurz mit Welle 13 bezeichnet wird. Die Energieversorgung kann über eine Batterie oder bei Vorsehen eines entsprechenden Anschlusses, direkt über das Stromnetz des Fahrzeugs, bspw. über den Zigarettenanzünder oder eine spezielle Steckdose, erfolgen.

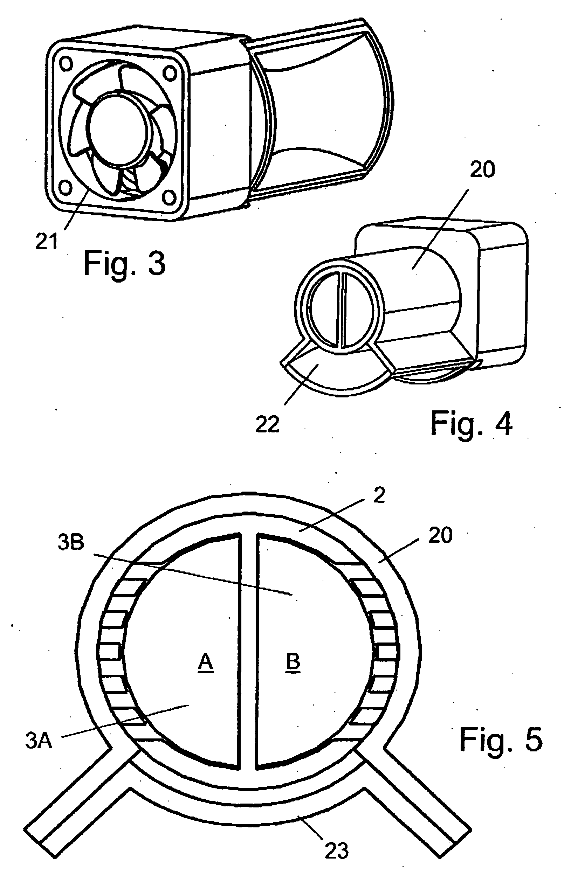

Um das Gebläse 10 sowie den inneren Hohlzylinder 2 ist ein Gehäuse 20 angeordnet, welches eine erste Öffnung 21 auf der Gebläseseite zum Einlassen der vom Lüfterrad 11 angesaugten Luft und eine zweite Öffnung 22 zum Auslassen der mit einem Duft versetzten Luft aufweist. Das Gehäuse 20 weist im Bereich des ersten Bauteils einen über 270° sich erstreckenden hohlzylindrischen Bereich auf, dessen längsseitige Enden in sich radial nach außen erstreckender Weise fortgeführt sind. In diesem Bereich ist eine Wandfläche 23 angeordnet, welche gebläseseitig und austrittsseitig die Enden außenseitig miteinander verbindet, während im mittleren Bereich die Wandfläche die Enden des hohlzylindrischen Bereichs miteinander verbindet, wobei der Übergang gekrümmt erfolgt, wie aus Fig. 3 ersichtlich. Durch den mittleren Bereich wird der direkte Durchgang versperrt, so dass die Luft im Falle einer Beduftung durch die erste Reihe von schlitzförmigen Eintrittsöffnungen in die entsprechende Aufnahmekammer 3 und durch die zweite Reihe von schlitzförmigen Austrittsöffnungen wieder heraus aus der Aufnahmekammer 3 und zur zweiten Öffnung 22 des Gehäuses gelangt, durch welche die nunmehr mit dem entsprechenden Duft A oder B versetzte Luft in den Fahrzeuginnenraum ausgelassen wird.

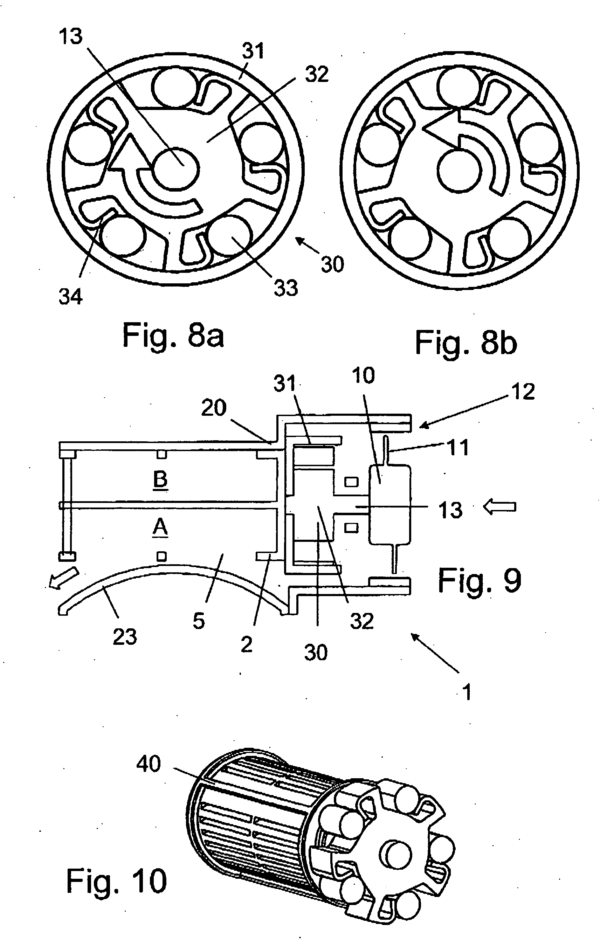

Um die Anordnung der Aufnahmekammern 3A und 3B des inneren Hohlzylinders 2 im Gehäuse 20 zu verändern, ist der innere Hohlzylinder 2 verdrehbar im Gehäuse 20 angeordnet, wobei er endseitig über einen Freilauf 30 mit der Welle 13 verbunden ist. Dreht sich der Motor 12 (und das Laufrad 11) in der Richtung, in welcher das Lüfterrad 11 Luft ansaugt und in das Gehäuse 20 fördert, so erfolgt durch den Freilauf 30 keine Kraftübertragung auf den inneren Hohlzylinder 2 (Fig. 8b). Ist jedoch in Folge eines Änderungswunsches in Bezug auf die Beduftung eine Stellbewegung des inneren Hohlzylinders 2 erforderlich, so wird die Drehrichtung des Motors umgekehrt, so dass der Freilauf 30 klemmt (siehe Fig. 8a) und die Welle nimmt so den inneren Hohlzylinder 2 mit, bis die Bewegung bei Erreichen der gewünschten Endstellung beendet wird, d.h. die Drehrichtung des Motors wieder umgekehrt wird.

Eine Rastung, bspw. gebildet durch federbelastete Kugeln, welche in Öffnungen an der Außenfläche des Freilaufs verrasten, oder eine federgelastete Sperrklinke in Verbindung mit Rastzähnen auf der Außenseite des Freilaufs kann zusätzlich die Position sichern und ein schleichendes Weiterdrehen im Lüfterbetrieb in Folge Reibung verhindern.

Hierbei ist der innere Hohlzylinder 2 auf der Gebläseseite starr mit einem äußeren Ring 31 des Freilaufs 30 verbunden, während der zentrale Bereich 32 des Freilaufs 30 starr mit der Welle 13 und somit dem Rotor des Motors 12 verbunden ist. Zwischen dem äußeren Ring 31 und dem zentralen Bereich 32 ist eine Mehrzahl von Klemmrollen 33 in Freiräumen angeordnet, welche bei der in Fig. 8a dargestellten Drehrichtung durch in Richtung eines verjüngten Endes der Freiräume gezwängt werden und daher eine Relativbewegung verhindern, d.h. der äußere Ring 31 wird von der Welle 13 mitgenommen, während bei der in Fig. 8b dargestellten Drehrichtung die Klemmrollen 33 aus diesen verjüngten Enden der Freiräume in Richtung des anderen Endes bewegt werden und in Anlage an Federarme 34 gelangen, so dass der äußere Ring 31 nicht weiter mitgenommen wird.

Der äußere Ring 31 ist mit einem Boden ausgebildet, welcher fest mit der einen Endfläche des inneren Hohlzylinders 2 verbunden ist, beispielsweise ist eine einstückige Ausgestaltung möglich. Zur Lagerung der Welle 13 ist in Verlängerung der Mittellängsachse des Hohlzylinders, konzentrisch im Boden des äußeren Rings 31 ein Lager angeordnet (siehe Fig. 9).

Der Zusammenbau von innerem Hohlzylinder 2 samt Freilauf 30 und Lüfterrad 11 in das Gehäuse 20 kann mittels Einschiebens von der Gebläseseite des Gehäuses 20 her erfolgen. Zur Fixierung im Gehäuse 20 können Schnapphaken vorgesehen sein, welche bspw. den äußeren Ring 31 des Freilaufs umgreifen und in Längsrichtung festhalten, jedoch eine Drehbewegung des Rings zulassen. Alternativ kann die Ausgestaltung auch mehrteilig sein, wobei beispielsweise der innere Hohlzylinder von der anderen Seite eingeschoben und beispielsweise mittels einer Clipsverbindung im Gehäuse fixiert werden kann.

An Stelle des zuvor beschriebenen Freilaufs 30 kann auch eine beliebige andere Ausgestaltung des Freilaufs vorgesehen sein, wobei beispielsweise auch der mit der Welle verbundene Bereich als äußeres Element und der je nach Drehrichtung der Welle angetriebene oder stehende Bereich als zentrales Element ausgebildet sein kann.

Zur Abdichtung kann bei einem zweiten, nicht in der Zeichnung dargestellten Ausführungsbeispiel, welches ansonsten entsprechend dem ersten Ausführungsbeispiel aufgebaut ist, auf der Außenseite des inneren Hohlzylinders ein Dichtelement angeordnet, welches vier in Längsrichtung verlaufende, äquidistant zueinander angeordnete Stege sowie endseitig zwei Ringe umfasst, wobei die Stege die einzelnen Bereiche mit Ein- und Austrittsöffnungen und die Zwischenbereiche ohne Öffnungen voneinander trennt. Die Stege verhindern somit ein Ausströmen von Duftstoff über die Spalte zwischen dem inneren Hohlzylinder und dem Gehäuse in Folge von Querströmungen in den Zwischenstellungen, in welchen ein Bereich ohne Öffnungen im Blendenbereich angeordnet ist, d.h. in welchen kein Dufteintrag in den Fahrzeuginnenraum gewünscht wird, oder ein teilweises Ausströmen des zweiten, auf der gegenüberliegenden Seite angeordneten Duftstoffs bei Wahl des ersten Duftstoffs und umgekehrt. Beispielhaft sind entsprechende Dichtelemente 40 im folgenden dritten Ausführungsbeispiel erläutert und in Fig. 10 dargestellt.

Gemäß dem dritten Ausführungsbeispiel, das in Fig. 10 dargestellt ist, weist der innere Hohlzylinder drei gleich große Bereiche auf, wobei in zwei derselben ein Duftstoff angeordnet ist und der dritte Bereich ohne einen Duftstoff versehen ist. Über den gesamten Umfang sind Schlitze als Ein- und Ausströmöffnungen vorgesehen. Beim Wechseln zwischen zwei unterschiedlichen Düften, die in den zwei Bereichen vorgesehen sind und bei entsprechender Ausbildung des Gehäuses 20, wird ein Bereich oder Sektor ohne Duftstoff in den Luftstrom gedreht. Somit lässt sich auch eine Stellung realisieren, in der trotz eingeschaltetem Gebläse 10 keine Beduftung erfolgt. Eine solche Neutralstellung kann nützlich sein um bei unbeabsichtigter Betätigung des Gebläses 10 einen nicht erwünschten Duftaustrag zu vermeiden. Um ein Überströmen von Luft in den benachbarten Bereich zu verhindern, ist ein Dichtelement 40 mit Stegen und zwei ringförmigen Endbereichen auf der Außenfläche des inneren Hohlzylinders vorgesehen. Das Dichtelement 40 besteht vorteilhafterweise aus einem elastomeren Kunststoff oder Gummi. Jeder Bereich oder Sektor ist somit gegenüber dem Gehäuse 20 abgedichtet. Auch in diesem Fall ist wieder ein Freilauf vorgesehen, welcher dem des ersten Ausführungsbeispiels entspricht. Ferner ist der Bereich, in welchem die gekrümmte Wandfläche vorgesehen ist, welche eine Wand des Luftkanals vom Gebläse zur Luftaustrittsöffnung bildet, über einen Winkel von annähernd 120° ausgebildet, d.h. sie ist etwas größer als im Falle der ersten beiden Ausführungsbeispiele.

Eine Ausgestaltung gemäß dem dritten Ausführungsbeispiel hat den kleinen Nachteil, dass - da nur eine Drehrichtung des inneren Hohlzylinders, in welchem die Duftstoffe angeordnet sind, möglich ist - im Rahmen einer Stellbewegung des inneren Hohlzylinders kurzfristig der zweite Duft freigesetzt werden kann, solange die Stellbewegung erfolgt und dieser Bereich im Bereich des Luftkanals angeordnet ist. Da dies jedoch nur kurzfristig der Fall ist, und zudem das Lüfterrad nur langsam in entgegengesetzter Richtung gedreht wird, wird nur wenig von diesem nicht gewünschten Duft freigesetzt. Will man diesen aufgezeigten Nachteil vermeiden, ist es möglich in einer weiteren, nicht in den Figuren dargestellten Ausführung beispielsweise vier Bereiche vorzusehen, wobei in Drehrichtung jeweils ein mit Duftstoff gefüllter Bereich und ein Bereich ohne Duftstoff aufeinanderfolgen.

Alternativ kann es auch gewünscht sein, dass es zu einer Vermischung der Duftstoffe kommt. So ist es möglich mittel der Anordnung gemäß dem dritten Ausführungsbeispiel nicht nur die "reinen" Duftnoten der beiden Bereiche zu realisieren, sondern bei geeigneter Wahl der beiden Duftstoffe auch einen Mischduft nach einem vorgegebenen Einstellverhältnis der beiden Einzeldüfte zu komponieren.

Die Regelung des Beduftungssystems 1 kann sowohl handbetätigt, getaktet als auch in Abhängigkeit von Messwerten, ermittelt über entsprechende Sensoren, wie Duftsensoren und/oder Temperaturfühler, und in Abhängigkeit der ermittelten Konzentration und/oder Güte der Luft erfolgen.

Die Ausgestaltung des einen oder mehrere Duftstoffe enthaltenden Teils des Beduftungssystems kann auch von den oben beschriebenen Ausführungsformen abweichen. Beispielsweise kann die Ausgestaltung entsprechend der in der nachveröffentlichten EP 07 009 533 A1 erfolgen, deren Offenbarungsgehalt in Bezug auf die relativ zueinander verdrehbaren Bauteile, nämlich dem inneren Hohlzylinder und dem Gehäuse, ausdrücklich mit einbezogen wird.

Gemäß einem weiteren, nicht in der Zeichnung dargestellten Ausführungsbeispiel sind an Stelle von zwei Duftstoffen, wie in der Zeichnung dargestellt, vier Duftstoffe in 45°-Sektoren angeordnet, wobei zwischen jedem Sektor ein Sektor ohne Duftstoff angeordnet ist. Alternativ können beispielsweise auch drei 60°-Sektoren oder sechs 30°-Sektoren vorgesehen sein.

## Patentansprüche

1. Beduftungssystem, insbesondere für ein Kraftfahrzeug, aufweisend mindestens zwei relativ zueinander verdrehbare Bauteile (2, 20), wobei in mindestens einer Relativstellung der Bauteile zueinander mindestens eine Öffnung (5) freigegeben ist, welche in mindestens einer anderen Relativstellung der Bauteile (2, 20) verschlossen ist, und mindestens ein von einem Motor (12) antreibbares Lüfterrad (11) eines Gebläses (10), welches auf der Motorwelle (13) sitzt oder mit derselben gekoppelt ist,
**dadurch gekennzeichnet, dass**
mindestens ein Freilauf (30) zwischen der Motorwelle (13) und den relativ zueinander verdrehbaren Bauteilen (2, 20) des Beduftungssystems (1) vorgesehen ist.

2. Beduftungssystem nach Anspruch 1, **dadurch gekennzeichnet, dass** der Motor (12) in seiner Drehrichtung umdrehbar ist.

3. Beduftungssystem nach Anspruch 1 oder 2, **dadurch gekennzeichnet, dass** als Bauteile ein erstes, im Wesentlichen hohl ausgebildetes Bauteil (2), welches mindestens eine Aufnahmekammer (3) für einen Duft (A, B) oder eine einen Duft enthaltende Patrone bildet, und als zweites Bauteil ein das erste Bauteil zumindest bereichsweise umgebendes Gehäuse (20) vorgesehen sind, wobei zwischen den beiden Bauteilen (2, 20) mindestens ein Luftkanal ausgebildet ist, welcher Luft zumindest bereichsweise in das hohl ausgebildete Bauteil (2) ein- und ausleitet oder an einer Öffnung desselben vorbeileitet.

4. Beduftungssystem nach Anspruch 3, **dadurch gekennzeichnet, dass** der Luftkanal, welcher zwischen dem ersten und zweiten Bauteil ausgebildet ist, einen sich in Strömungsrichtung veränderlichen Querschnitt zwischen den beiden Bauteilen aufweist, wobei sich der Strömungsquerschnitt ausgehend von einem maximalen Einströmquerschnitt auf Gebläseseite entlang der Längsrichtung des Kanals zuerst allmählich verringert, bis er im mittleren Bereich minimal ist, insbesondere keine freie Querschnittsfläche zwischen den beiden Bauteilen vorhanden ist, und sich anschließend wieder erweitert, insbesondere wieder auf eine Größe, welche der des Einströmquerschnitts entspricht.

5. Beduftungssystem nach einem der vorhergehenden Ansprüche, **dadurch gekennzeichnet, dass** eine starre Koppelung zwischen Motor und einem Lüfterrad (11) des Gebläses (10) vorgesehen ist, wobei sich die Drehrichtung des Lüfterrades (11) im Falle einer Stellbewegung der beiden relativ zueinander verdrehbaren Bauteile umdreht.

6. Beduftungssystem nach einem der Ansprüche 1 bis 4, **dadurch gekennzeichnet, dass** zwischen dem Motor und einem Lüfterrad (11) des Gebläses (10) ein zweiter Freilauf vorgesehen ist, welcher in entgegengesetzter Richtungen zum ersten Freilauf (30) eine Relativbewegung freigibt oder das Lüfterrad (11) an die Welle, insbesondere Motorwelle, ankoppelt.

7. Beduftungssystem nach einem der vorhergehenden Ansprüche, **dadurch gekennzeichnet, dass** der Motor in das Gebläse (10) integriert ist.

8. Beduftungssystem nach einem der vorhergehenden Ansprüche, **dadurch gekennzeichnet, dass** das erste Bauteil (2), welches relativ zu einem zweiten Bauteil (20) verdrehbar ist, starr, insbesondere einstückig, mit einem abtriebsseitigen Teil des Freilaufs (30) verbunden ist.

9. Beduftungssystem nach einem der vorhergehenden Ansprüche, **dadurch gekennzeichnet, dass** konzentrisch im abtriebsseitigen Teil des Freilaufs (30) der antriebsseitige Teil des Freilaufs (30) drehbar gelagert ist.

10. Verfahren zum Betreiben eines Beduftungssystems (1) nach einem der Ansprüche 1 bis 9, **gekennzeichnet durch** eine Richtungsumkehr der Drehrichtung des Motors (13) zur Verstellung der Position der relativ zueinander verdrehbaren Bauteile.

## Claims

1. Fragrance system, in particular for a motor vehicle, having at least two components (2, 20) rotatable relative to each other, wherein at least one opening (5) is released in at least one relative position of the components to each other, which opening is closed in at least another relative position of the components (2, 20), and at least one blower wheel (11) of a blower (10), which can be driven by a motor (12), which blower sits on the motor shaft (13) or is coupled thereto,
**characterised in that**
at least one free-wheel (30) is provided between the motor shaft (13) and the components (2, 20) of the fragrance system (1) rotatable relative to each other.

2. Fragrance system according to claim 1, **characterised in that** the motor (12) can be reversed in its rotary direction.

3. Fragrance system according to claim 1 or 2, **characterised in that** a first, essentially hollow component (2), which forms at least one reception chamber (3) for a fragrance (A, B) or a cartridge containing a fragrance, and a second component which forms a housing (20) surrounding the first component at least in sections, are provided as components, wherein at least one air channel is formed between the two components (2, 20), which channel guides air into and out of the hollow component (2) at least in sections or guides it past an opening thereof.

4. Fragrance system according to claim 3, **characterised in that** the air channel which is formed between the first and the second component, has a cross section between the two components which can be changed in the flow direction, wherein the flow cross section first gradually reduces starting from a maximum inflow cross section on the blower side along the longitudinal direction of the channel, until it is minimal in the centre region, especially no free cross sectional surface is present between the two components, and subsequently widens again, especially again to a size which corresponds to the one of the inflow cross section.

5. Fragrance system according to one of the preceding claims, **characterised in that** a rigid coupling between the motor and a blower wheel (11) of the blower (10) is provided, wherein the rotary direction of the blower wheel (11) reverses in the case of an adjusting movement of the two components which can be rotated relative to each other.

6. Fragrance system according to one of claims 1 to 4, **characterised in that** a second free-wheel is provided between the motor and a blower wheel (11) of the blower (10), which free-wheel releases a relative movement in the opposite direction to the first free-wheel or couples the blower wheel (11) to the shaft, particularly the motor shaft.

7. Fragrance system according to one of the preceding claims, **characterised in that** the motor is integrated in the blower (10).

8. Fragrance system according to one of the preceding claims, **characterised in that** the first component (2), which is rotatable relative to a second component (20), is connected to the output part of the free-wheel (30) in a rigid manner, particularly in one piece.

9. Fragrance system according to one of the preceding claims, **characterised in that** the part of the free-wheel (30) on the drive side is mounted concentrically in the part of the free-wheel (30) on the output side in a rotatable manner.

10. Method for operating a fragrance system (1) according to one of claims 1 to 9, **characterised by** a direction reversal of the rotary direction of the motor (13) for adjusting the position of the components which can be rotated relative to each other.

## Revendications

1. Système servant à parfumer, en particulier prévu pour un véhicule automobile, présentant au moins deux composants (2, 20) pouvant être déplacés en torsion l'un par rapport à l'autre, où au moins une ouverture (5) est libérée dans au moins une position relative des composants l'un par rapport à l'autre, ouverture qui est fermée dans au moins une autre position relative des composants (2, 20), et présentant au moins une roue de ventilateur (11) d'une soufflante (10), entraînée par un moteur (12), soufflante qui est montée sur l'arbre (13) du moteur ou est couplée à celui-ci,
**caractérisé en ce qu'**il est prévu au moins une roue libre (30) entre l'arbre (13) du moteur et les composants (2, 20) du système (1) servant à parfumer, lesdits composants pouvant être déplacés en torsion l'un par rapport à l'autre

2. Système servant à parfumer selon la revendication 1, **caractérisé en ce que** le moteur (12) peut être inversé dans son sens de rotation.

3. Système servant à parfumer selon la revendication 1 ou 2, **caractérisé en ce qu'**il est prévu, comme composants, un premier composant (2) configuré sensiblement de façon creuse, composant qui forme au moins une chambre réservoir (3) pour un parfum (A, B) ou une cartouche contenant un parfum et, comme deuxième composant, un carter (20) entourant au moins partiellement le premier composant, où au moins un conduit d'air est configuré entre les deux composants (2, 20), conduit d'air qui fait entrer et sortir de l'air, au moins partiellement, dans et hors du composant (2) configuré de façon creuse, ou bien fait passer de l'air devant une ouverture de ce composant.

4. Système servant à parfumer selon la revendication 3, **caractérisé en ce que** le conduit d'air, qui est formé entre le premier et le deuxième composant, présente, entre les deux composants, une section se modifiant dans la direction d'écoulement, où la section d'écoulement, à partir d'une section d'écoulement d'entrée maximale, diminue d'abord progressivement, sur le côté soufflante, le long de la direction longitudinale du conduit, jusqu'à ce que la section d'écoulement soit minimale dans la zone centrale, en particulier qu'il n'existe aucune surface de section libre entre les deux composants et, ensuite, qu'elle s'élargisse à nouveau, en particulier pour atteindre une nouvelle fois une dimension qui corresponde à celle de la section d'écoulement d'entrée.

5. Système servant à parfumer selon l'une quelconque des revendications précédentes, **caractérisé en ce qu'**il est prévu un couplage fixe entre le moteur et une roue de ventilateur (11) de la soufflante (10), où la direction de rotation de la roue de ventilateur (11) s'inverse dans le cas d'un mouvement de réglage des deux composants pouvant être déplacés en torsion l'un par rapport à l'autre.

6. Système servant à parfumer selon l'une quelconque des revendications 1 à 4, **caractérisé en ce qu'**il est prévu une deuxième roue libre entre le moteur et une roue de ventilateur (11) de la soufflante (10), deuxième roue libre qui libère un mouvement relatif par rapport à la première roue libre (30), suivant des directions opposées, ou bien couple la roue de ventilateur (11) à l'arbre, en particulier à l'arbre du moteur.

7. Système servant à parfumer selon l'une quelconque des revendications précédentes, **caractérisé en ce que** le moteur est intégré dans la soufflante (10).

8. Système servant à parfumer selon l'une quelconque des revendications précédentes, **caractérisé en ce que** le premier composant (2), qui peut être déplacé en torsion par rapport à un deuxième composant (20), est relié fixement, en particulier en formant une seule et même pièce avec une partie, côté sortie, de la roue libre (30).

9. Système servant à parfumer selon l'une quelconque des revendications précédentes, **caractérisé en ce que** la partie, côté entraînement, de la roue libre (30) est montée en rotation, de façon concentrique, dans la partie, côté sortie, de la roue libre (30).

10. Procédé de fonctionnement d'un système (1) servant à parfumer selon l'une quelconque des revendications 1 à 9, **caractérisé par** une inversion de direction du sens de rotation du moteur (13), servant au réglage de la position des deux composants pouvant être déplacés en torsion l'un par rapport à l'autre.
